# EUROPÄISCHE PATENTSCHRIFT

(11) **EP 1 513 445 B1**
(45) Veröffentlichungstag und Bekanntmachung des Hinweises auf die Patenterteilung: **06.09.2006**
(21) Anmeldenummer: 03735582.3
(22) Anmeldetag: 07.06.2003
(51) Int. Cl.: A61B 5/103

(54) **PNEUMATISCHES HAUTELASTIZITÄTSMESSGERÄT**
PNEUMATIC DEVICE FOR MEASURING SKIN ELASTICITY
APPAREIL PNEUMATIQUE DE MESURE DE L'ELASTICITE DE LA PEAU

(30) Priorität: 14.06.2002 DE 10226708
(43) Veröffentlichungstag der Anmeldung: 16.03.2005
(73) Patentinhaber: Courage + Khazaka electronic GmbH, 50829 Köln (DE)
(72) Erfinder: HEINRICH, Thorsten, 21698 Harsefeld (DE); LUNDERSTÄDT, Reinhart, 22885 Barsbüttel (DE); JASPERS, Sören, 22869 Schenefeld (DE); KOOP, Urte, 20149 Hamburg (DE); SCHREINER, Volker, 20259 Hamburg (DE); ENNEN, Joachim, 22397 Hamburg (DE)
(74) Vertreter: Dallmeyer, Georg
(86) Internationale Anmeldenummer: PCT/EP2003/006017
(87) Internationale Veröffentlichungsnummer: WO 2003/105689

(56) Entgegenhaltungen:
- US-A- 4 976 272
- US-A- 5 054 502
- US-A- 5 379 235
- HEINRICH AND LUNDERSTÄDT: "Quantification of mechanical properties of human skin in vivo" PROC. OF SPIE, APPLICATIONS OF DIGITAL IMAGE PROCESSING, Bd. 4472, - 2001 Seiten 11-20, XP008022210 in der Anmeldung erwähnt

## Beschreibung

Die Erfindung bezieht sich auf ein Messgerät und ein Verfahren zur Bestimmung bestimmter mechanischer Eigenschaften eines elastischen und/oder dehnbaren Materials, insbesondere der Haut, wobei mittels Unterdruck ein ausgewählter Bereich des Materials gespannt bzw. entspannt wird.

Mit fast zwei Quadratmetern Fläche und ca. 15 % des Körpergewichts ist die Haut nicht nur das größte, sondern auch eines der erstaunlichsten Organe unseres Körpers. Sie schützt die inneren Organe gegen Verletzung und gegen das Eindringen von Bakterien, reguliert die Körpertemperatur und vermittelt uns als Tastorgan sinnliche Eindrücke und Erfahrungen.

Jeder Quadratzentimeter unserer Haut besteht aus ungefähr
- 600.000 Zellen,
- 5.000 Rezeptoren zum Tasten und Fühlen von Temperatur, Druck, Schmerz, Berührungen,
- 100 Schweißdrüsen,
- 20 Talgdrüsen,
- 4 Metern Nervensträngen und
- einem Meter Adern.

Allgemein besteht die Haut (Cutis) aus der Oberhaut (Epidermis), einem mehrschichtigen verhornten Plattenepithel, und der Lederhaut (Dermis, Corium) mit einem engen Geflecht von Fasern, die fest mit der Oberhaut verflochten sind. Darunter schließt sich die Unterhaut (Tela subcutanea) an.

Die Oberhaut ist die Hautschicht, die mit einer Homschicht nach außen hin abschließt. In ihrer Keimschicht - wo übrigens auch die Hautpigmente sitzen - entstehen durch Zellteilung ständig neue Hautzellen, die in 28 Tagen an die Hautoberfläche wandern, wo sie absterben und als Schuppen abgestoßen werden. So erneuert sich die Epidermis alle 28 Tage.

Erheblich dicker als die Oberhaut ist die darunter liegende Lederhaut. Als Lieferant wichtiger Nährstoffe für die Oberhaut ist die Lederhaut mit Nerven, Blut- und Lymphgefäßen durchsetzt und besteht zu einem großen Teil aus faserigem Bindegewebe. Die Beschaffenheit dieses Bindegewebes bestimmt die Elastizität und Struktur der Haut. Haarwurzeln, Schweiß- und Talgdrüsen sitzen in der Lederhaut.

Die Unterhaut ist die Fortsetzung der Lederhaut, unterscheidet sich von dieser jedoch in ihrem Aufbau. Wie der Name vermuten lässt, ist die Lederhaut fest und faserig, während die Unterhaut gröber und eher schwammig ist. Sie besteht vorwiegend aus Binde- und Fettgewebe, das dem Körper u. a. als Kälteschutz und Energiespeicher dient. In diesen Zellen können größere Mengen Fett eingelagert werden, was zu Cellulite (besonders betroffen sind Frauen) führen kann.

Die in den Schichten des verhornten Plattenepithels vorkommenden Pigmentzellen (Melanozyten) sind für die Hautfarbe verantwortlich. Die Melaninbildung kann durch Sonnenstrahlen verstärkt werden ― Sonnenbräune. Vitamin A-Mangel führt zu vermehrter Verhornung (Hyperkeratose). Erst durch die Lederhaut erreicht die Haut ihre Festigkeit und Elastizität. Die oberen Anteile reichen zapfenförmig in die Oberhaut ― die Papillarschicht (Papillarkörper, Stratum papillare). In ihr befinden sich neben den Kollagenfasern die Blut- und Lymphkapillare sowie die Nervenaufzweigungen und spezielle Sinnesorgane (Meißner-Tastkönperchen). Die Geflechtsschicht (Stratum reticulare) darunter sorgt für die Elastizität der Haut. In ihr befinden sich die Haarwurzeln mit dem Haarmuskel (M. arrector pili), den Knäueln der Talgdrüsen sowie Vater-Pacini-Lamellenkörperchen. Die Unterhaut (Subcutis) ist mit gekammertem Binde- und Fettgewebe ausgefüllt und stellt die Verbindung zur oberflächlichen Körperfaszie her. In der Unterhaut befinden sich die größeren Blut- und Lyphgefäße. Die Haare (Pili) dienen meist der Tastempfindung und als Wärmeschutz. Die Nägel (Ungues) sind besondere Hornbildungen der Haut.

Die Oberhaut und Lederhaut zusammen messen an ihren dicksten Stellen - Rücken, Fußsohlen und Handflächen - 3 bis 5 mm, an der dünnsten Stelle, den Augenlidern, hingegen weniger als 1 Millimeter.

Die Elastizität der Gewebe verändert sich mit dem Alter. Sie wird durch verschiedene andere Parameter, beispielsweise Gesundheit, Ermüdungszustände, Einfluss äußerer Faktoren und dergleichen, beeinflusst.
Bereits ab dem 25. Lebensjahr verlangsamt sich die Zellerneuerung der Haut. Das Kollagen (quellende Eiweißkörper, wichtiger Bestandteil des Bindegewebes) kann weniger Wasser einlagern, da sich die Zusammensetzung im Laufe der Hautalterung verändert, und die Anzahl der Talg- und Schweißdrüsen nimmt ab. Dadurch wird die Oberhaut dünner und verliert zunehmend an Elastizität. Schlechtere Hautdurchblutung führt zu Feuchtigkeitsverlusten, das Hautgewebe erscheint nicht mehr straff.
Im Vergleich zum gesunden jungen Menschen treten an der Haut des alternden Menschen folgende Veränderungen auf :
- Es kann zu einer zunehmenden Verdünnung der obersten Hautschicht, der Oberhaut oder Epidermis, kommen.
- Epidermis und Dermis bilden immer weniger eine kompakte Einheit. Dadurch wird die alternde Haut anfälliger auch schon für kleinere Traumen und Scherbeanspruchungen.
- Die Dermis wird immer dünner und weniger elastisch.
- Die Dermis weist immer weniger Flbroblasten auf. Dadurch kommt es zu einer sinkenden Produktion von :
   - Elastin, das für die Elastizität der Haut verantwortlich ist und
   - Kollagen, das der Haut ihre Widerstandsfähigkeit, verleiht. Somit nehmen beim alten Menschen die Anfälligkeit für Scherbeanspruchungen und die Faltenbildung zu.
- Die Altershaut ist trocken (Xerosis).
- Geschädigte Hautpartien werden viel langsamer erneuert.
- Die Zahl der für die Immunabwehr wichtigen spezialisierten "Wächterzellen" (der sogenannten "Langerhans-Zellen") nimmt ab.
- An den Handrücken können sich homartige Wucherungen bilden (Keratosis senilis).
- Infolge der zunehmenden Beeinträchtigung der Melanozytenfunktion bräunt der alte Mensch nicht mehr so leicht. Was dabei aber schwerer wiegt, ist die Tatsache, daß dadurch die Haut alter Menschen weniger gut als die jüngerer gegen den schädlichen Wirkungen der Sonnenstrahlen geschützt ist und somit das Hautkrebsrisiko steigt.
- Es entwickeln sich Bereiche mit Hyperpigmentation Infolge von Melaninanhäufungen, etwa im Gesicht (Teleangiektasien).
- Bei vielen alten Menschen finden sich Hautblutungen bzw. braune Flecken an Händen und Armen (Purpura senilis). Dies ist vermutlich darauf zurückzuführen, dass die mangelnde Elastizität der Altershaut die Hautkapillaren verletzungsanfälliger macht und dass es zu einer Verdickung und Versteifung der Gefäßwände kommt.
Infolge von Lichteinwirkung treten folgende Veränderung der Haut auf:
- vermehrte Melaninbildung in der Epidermis. Es kommt zunächst zu einer Verdickung der Epidermis, sodann zu einer Atrophierung,
- Faltenbildung,
- deutliche Abnahme der Zahl der Langerhans-Zellen,
- signifikante Verminderung des Kollagens und
- chronische Sonnenexposition ab der Jugend bewirkt, dass im Alter ein erhöhtes Hautkrebs-Risiko gegeben ist.

Die Erhöhung der Elastizität und der Feuchtigkeit der Haut und somit die Wiederherstellung des jugendlichen Aussehens und die Verhinderung der Faltenbildung ist daher eines der Hauptziele der therapeutischen Kosmetik.

Es ist bekannt, zur Bestimmung der Eigenschaften oder Wirkungen bestimmter dermatoiogischer Behandlungen, bestimmte spezifische Eigenschaften der Haut, ihre Entwicklung im Laufe der Zeit und gegebenenfalls die eventuell bestehenden Beziehungen der Eigenschaften zueinander zu untersuchen.

Eines der wesentlichen Merkmale der Haut ist die Talgabsonderung pro Flächeneinheit, und zu ihrer Bestimmung sind bereits verschiedene Messvorrichtungen vorgeschlagen worden. Eine weitere wesentliche Eigenschaft ist ihre Elastizität, die durch Bestimmung des Elastizitätsmodul (Youngscher Modul) und anderer Parameter festgestellt werden kann.

Durch die Erfindung wird nun eine Meßmethode zur Verfügung gestellt, wodurch es möglich ist, die Elastizität der menschlichen Haut in vivo vor und nach der Aufbringung von bestimmten Zubereitungen unter Berücksichtigung und Erfassung der Vorzugsrichtung zu bestimmen, wodurch sowohl ein objektiver Test für die Wirksamkeit solcher Zubereitungen, als auch ein Test hinsichtlich der Wirkung auf die damit behandelte Person, erhalten wird.

Im Sinne der Erfindung ist als Haut auch ein folienartiges Substrat zu verstehen, dessen elastische Eigenschaften durch das erfindungsgemäße Messgerät bestimmbar sind. Folienartige Substrate sind zum Beispiel dünne Polymerfolien, die eine gewisse Dehnung erfahren können. Auch sie können durch Einflüsse, die durch das Produktionsverfahren gegeben sind, ein anisotropes Dehnungs- und Streckverhalten aufweisen, weiches sich durch das unten beschriebene Messgerät bestimmen lässt.

Den derzeit in der kosmetischen Industrie am weitesten verbreiteten Ansatz, um die Hautoberfläche einer messtechnischen Erfassung zugänglich zu machen, stellt die Anfertigung von Silikon-Negativabdrücken - sogenannten Replikaten - dar. Diese werden dann stellvertretend taktil oder optisch vermessen. Die Anfertigung der Replikate erweist sich jedoch insbesondere bei hohen Probandenzahlen als sehr zeitaufwendig und ist immer mit einem in aller Regel unbekannten informationsverlust verbunden.

Die Bestimmung von mechanischen Eigenschaften der Haut in zwei Dimensionen wird in dem Artikel "Two-dimensional elastic properties of human skin in terms of an incremental model at the in vivo configuration" von Reishner, Balogh und Menzel (Med.Eng.Phys., 1995, Vol. 17, 304-313) dargestellt. Die Messungen finden hier an Hautproben, die einem Probanden entnommen wurden, statt. Eine in vivo Messung ist nicht vorgesehen, bzw. ist nach der In diesem Artikel offenbarten Meßmethode nicht möglich.

Aus Ullstein Lexikon der Medizin (Ullsteinverlag 1970, Seiten 590 und 674) ist es in der Medizin bekannt, eine Vakuumquelle zu verwenden, um auf das menschliche Gewebe eine Saugwirkung zu erzielen, wobei es sich hier um eine Saugglocke zum Absaugen von Abszessen und einen Vakuumextraktor zur Verwendung in der Geburtshilfe handelt.

In DE 29 09 092 wird eine Methode zur Messung der Hautelastizität beschrieben, bei der - angelehnt an die üblichen Prüfmethoden der Werkstoffprüfung - ein Taster mit einem bestimmten Druck auf die Haut aufgebracht wird und abhängig von der Eindringtiefe die Hautelastizität bestimmt wird.

Die Messung der mechanischen Eigenschaften der Haut in vivo wird gegenwärtig auf dem Gebiet der kosmetischen wie der medizinischen Dermatologie vorwiegend mittels vier verschiedener Geräte bzw. Verfahren durchgeführt.

Einige Verfahren am Markt sind:
- das Extensometer: Dehnung der Haut mittels zwei auf der Haut aufgesetzter Messfühler, wobei die Dehnung (Änderung des Abstandes zwischen den Messfühlern) in Abhängigkeit der angelegten Kraft gemessen wird.
- das Torquemeter (Fa. Diastron Ltd., UK): Auf die Hautoberfläche wird hierbei eine innere Scheibe und ein äußerer Haltering verklebt. Mittels der Scheibe wird ein Drehmoment angelegt und der Winkel der dann erfolgten Auslenkung gegenüber dem Halteringgemessen. Danach stoppt die Krafteinleitung und die Scheibe kehrt infolge der Hautelastizität nahezu in die Ausgangslage zurück. Aufgrund der Komplexität der Auslenkung, namentlich durch die Verknüpfung von Normal- und Torsionsspannungen, ist eine Interpretation der erhaltenen Meßdaten hinsichtlich ihrer Relevanz zur Beurteilung von einzelnen Hautkomponenten ausgesprochen problematisch.
- das Cutometer (Fa. Courage Khazaka, Köln, Deutschland): Die Meßsonde wird bei diesem Gerät senkrecht auf die Haut gesetzt, die Meßsonde hatan der Unterseite eine Öffnung. Ein Unterdruck wird angelegt und die Haut wird in die Öffnung hineingezogen. In der Meßsonde wird das Maß der Auslenkung optoelektronisch gemessen. Auch in diesem Verfahren erfolgt keine tangentiale Auslenkung der Haut, so dass die dämpfenden Elemente des dermalen Gewebes sowie des subkutanen Fettgewebes vornehmlich in die Messung eingehen.
- das Ballistömeter: Bestimmung der Hautelastizität durch Messung des Dämpfungs- bzw. Schwingverhaltens der Haut bei Anregung mit einem Gewicht (DE 27 32 836), wobei ein oberflächenparallel aufgehängtes Pendel auf die Hautoberfläche fallengelassen und die Dämpfung der Schwingung aufgezeichnet wird. Da Schwingungsdämpfungen stets eine Funktion viskoser Elemente ist, muß vermutet werden, gerade hinsichtlich der Krafteinwirkung in Richtung des Normalenvektors, dass vor allem die Eigenschaften der Subkutis und weniger der Dermis und Epidermis in die Meßergebnisse eingehen.

Keines der aufgeführten Geräte ist zur Messung in vivo in der Lage, die Vorzugsrichtungen der Hautelastizität, deren Existenz der Medizin und der humanblologischen Forschung lange bekannt sind, zu detektieren und bei der Erhebung der Meßparameter zu berücksichtigen.

Das Multiaxiale-Extensometer ist eine Weiterentwicklung des Extensometers, bei der das Dehnungsverhalten in mehreren Richtungen simultan erfasst wird (DE 197 19 336). Leider hat auch hier der Kleber, der zur Kopplung der Haut an das Messgerät benötigt wird, einen zum Teil unbekannten Einfluss auf die gemessenen Deformationen.
Ein weiterer Nachteil ist, dass als Kleber Acrylate (Sekundenkleber) oder doppelseitige Klebebänder verwendet werden, die sehr schwer entfernbar sind und zu starken Hautreizungen neigen.

Aufgabe der im folgenden beschriebenen Erfindung ist es, eine Vorrichtung und ein Verfahren zur Verfügung zu stellen, mit dem präzise und reproduzierbare Messergebnisse über die Hautelastizität erzielbar sind. Des weiteren ist es ein Ziel der Erfindung, eine multiaxiale, definierte Belastung der Haut bei gleichzeitiger sensorischer Erfassung der jeweils auftretenden Dehnungen zu ermöglichen (Retardationsversuch), und darüber hinaus eine exakte Diskriminierung und Lokalisiervng der elastischen Vorzugsrichtungen der Haut (Langer'sche Linien) zuzulassen.

Gelöst wird diese Aufgabe durch ein Hautelastizitätsmessgerät wie es in Anspruch 1 gekennzeichnet ist. Das Messverfahren ist dabei Gegenstand der Unteransprüche.

Die erfindungsgemäße Vorrichtung enthält eine mechanische Einrichtung zur Streckung der Haut und hat vorzugsweise eine optoelektronische Auswertungseinrichtung.

Die Bilder 1 bis 3 zeigen einen erfindungsgemäßen Aufbau und die Bilder 4 bis 5 verdeutlichen die Funktion des Hautelastizitätsmessgeräts (HEMG). Die Darstellungen werden im folgenden erläutert.

Es zeigen
- Abbildung 1: den prinzipiellen Aufbau des Messgeräts in Verbindung mit den Prozess- und Signalleitwegen, durch die die Einzelkomponenten in Verbindung stehen;
- Abbildung 2: einen nicht maßstabsgetreue schematische Seitenansicht einer Ausführungsform eines erfindungsgemäßen Messkopfes;
- Abbildung 3: einen nicht maßstabsgetreue schematische Vorderansicht einer Ausführungsform eines erfindungsgemäßen Messkopfes;
- Abbildungen 4a bis 4c: einen nicht maßstabsgetreuen schematischen Querschnitt einer Ausführungsform eines erfindungsgemäßen Saugringes;
- Abbildungen 5a bis 5c: die schematische Darstellung der Prozessschritte vor (5a), bei (5b) und nach (5c) Hautdehnung durch Anlegen eines Unterdrucks;
- Abbildung 6: die Gegenüberstellung der Größen Druck, Triggersignal und Dehnung, wie sie In Abhängigkeit der Messzeit gewonnen werden;
- Abbildung 7: die Schematische Darstellung der Hautdehnung als Verschiebungsvektorfeld bei uniaxialer Dehnung;
- Abbildung 8: die Schematische Darstellung der Hautdehnung als Verschiebungsvektorfeld bei multiaxialer Dehnung und
- Abbildung 9: die Darstellung der Beträge der Hautdehnung als Graustufenbild bei multiaxialer Dehnung.

### Aufbau:

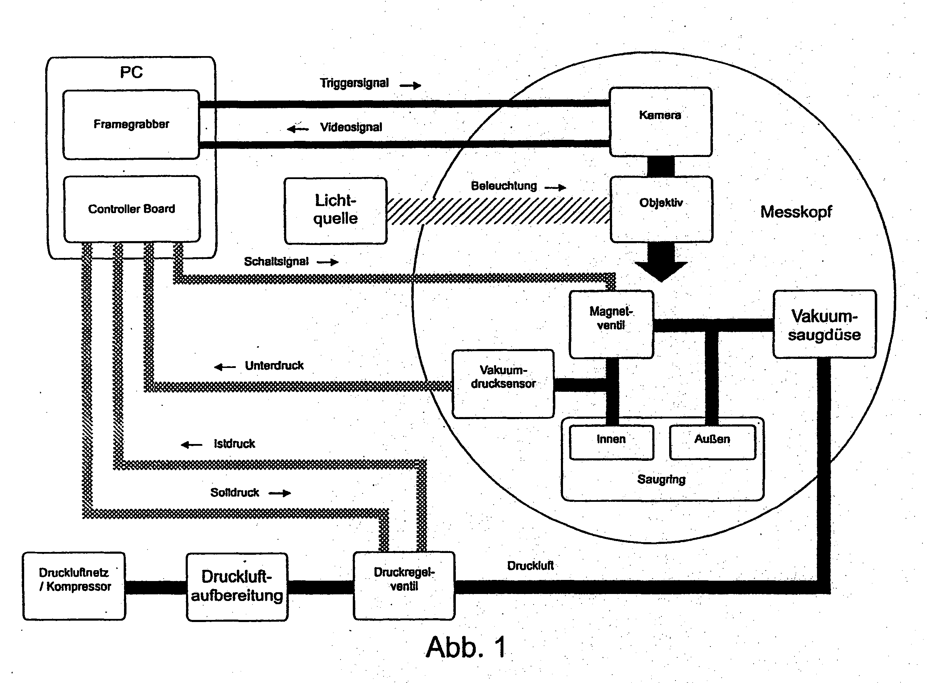

Im folgenden wird der Aufbau des Hautelastizitätsmessgerätes - im weiteren Text mit HEMG bezeichnet - anhand der Abbildungen 1 bis 4 näher erläutert, wobei hierdurch die Erfindung nicht beschränkt werden soll.

Abbildung 1 gibt eine Übersicht über die im HEMG enthaltenen Komponenten und deren Zusammenwirken. Aus Gründen der Übersichtlichkeit ist die Spannungsversorgung der elektrisch betriebenen Elemente nicht dargestellt. Das HEMG besteht aus vier wesentlichen Gruppen:
- einer Mess- und Steuereinheit (PC),
- einer Vakuumbereitstellung,
- einer Lichtquelle und
- einem Messkopf.

Die Mess- und Steuereinheit übernimmt die Versuchssteuerung, die Aufnahme und Speicherung der Messwerte (Daten), die Auswertung der Daten sowie die Darstellung von Ergebnissen.
Hierzu ist die Mess- und Steuereinheit neben den herkömmlichen Komponenten mit einem Framegrabber und einem Controller Board mit digitalen und analogen Ein- und Ausgängen ausgestattet.
Bevorzugt ist die Verwendung einer mikroprozessorgesteuerten Datenverarbeitungsanlage. Besonders geeignet sind programmierbare digitale Datenverarbeitungsanlagen.

Die Vakuumbereitstellung erfolgt durch Druckluft, wobei ein elektrisch angesteuertes Proportional-Druckregelventil den Druck, der die Saugdüse durchströmenden Luft, regelt. Dem Druckregler vorgeschaltet ist eine Druckluftaufbereitung, die ein Handeinschaltventil, einen Partikelfilter und ein manuelles Druckregelventil zum Einstellen des Reglereingangsdrucks enthalten kann.

Bevorzugt ist ebenfalls der Einsatz von Vakuumpumpen, insbesondere geregelten Vakuumpumpen.

Die Lichtquelle liefert das Licht zur telezentrischen Auflichtbeleuchtung des Messobjekts. Erfindungsgemäße Lichtquellen stellen Licht im sichtbaren Bereich zur Verfügung. Für bestimmte Messreihen ist der Einsatz von Lichtquellen möglich, die polarisiertes Licht oder Licht bestimmter Wellenlänge aussenden. Auch die Verwendung von Strahlung außerhalb des sichtbaren Bereiches kann von Vorteil sein.
Besonders geeignet sind Kaltlichtquellen, die über flexible Lichtleiter eine genau positionierbare Ausleuchtung ermöglichen.

Eine Ausführungsform eines erfindungsgemäßen Messkopfes ist in Bild 2 und 3 als Konstruktionszeichnung dargestellt, wobei hierdurch die Erfindung nicht beschränkt werden soll.

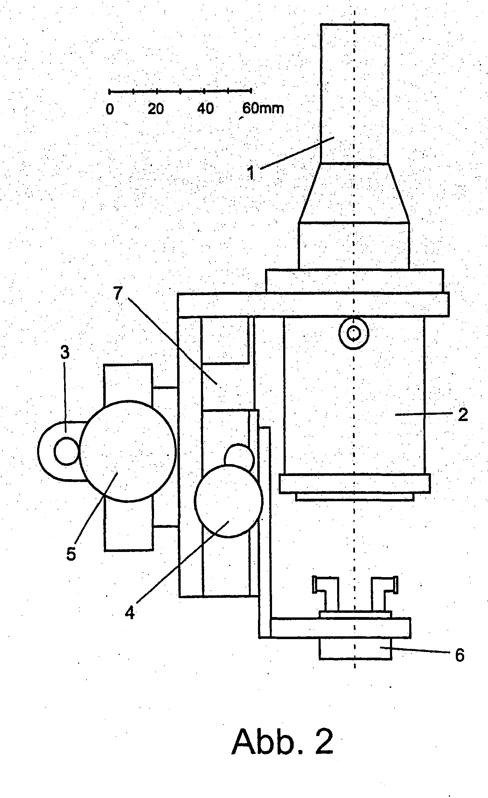

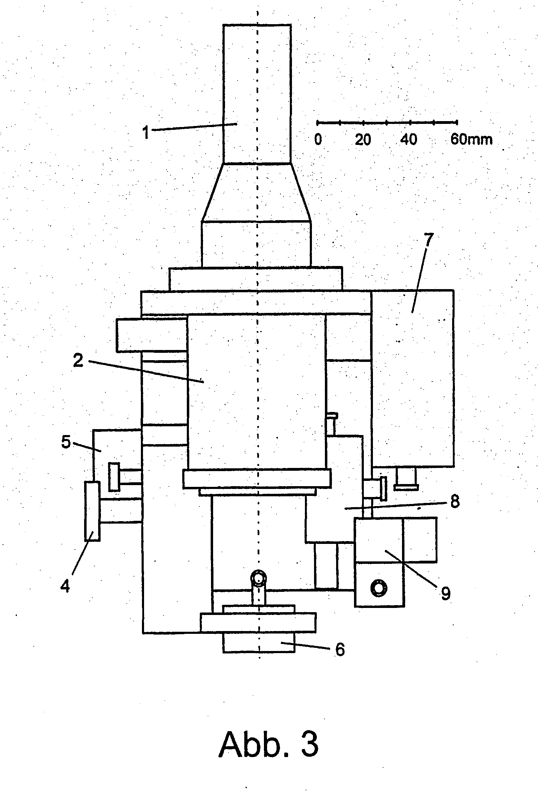

Der Messkopf dient der eigentlichen Durchführung der Messung. Er ist am Befestigungspunkt (3) an einem flexiblen Stativ (in Abb. 2 und 3 nicht wiedergegeben) befestigt und wird damit über dem zu untersuchenden Areal positioniert. Er enthält nur die Komponenten, die in der unmittelbaren Nähe des Messobjekts benötigt werden. Damit das während eines Drucksprungs zu evakuierende Volumen klein bleibt, zählen hierzu auch Saugdüse (8), Magnetventil (9) und Drucksensor (7). Die Versorgungsleitungen (Kabel, Lichtleiter, Druckluftschläuche) sind der besseren Übersichtlichkeit wegen in den Abb. 2 und 3 nicht dargestellt.

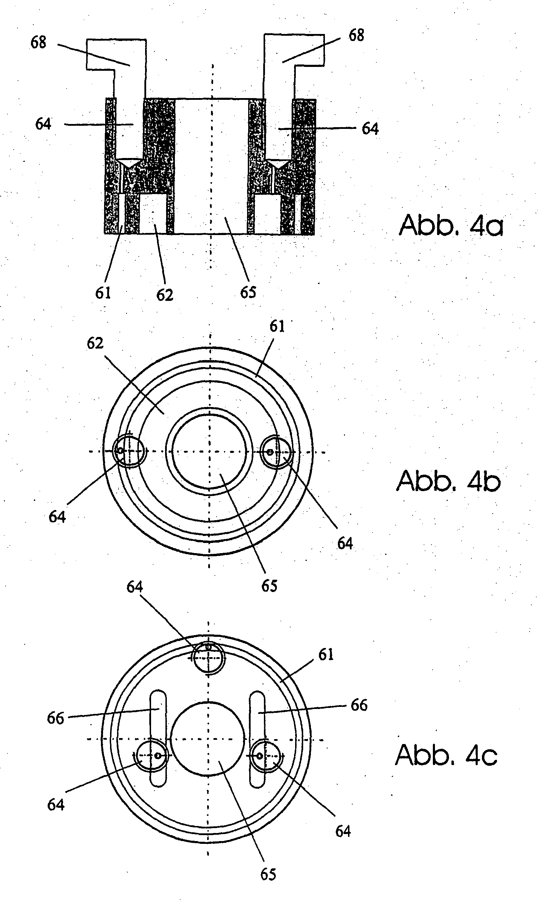

Der Messkopf beinhaltet neben weiteren Teilen folgende Elemente (Abbildung 2 und 3):
(1) Digitalkamera (Progressive Scan Kamera,)
(2) Telezentrisches Messobjektiv mit integrierter Einrichtung für telezentrische Auflichtbeleuchtung zur optischen Erfassung des Messobjekts im innenloch des Saugrings,
(3) Befestigungspunkt zur Ankopplung an das Stativ,
(4) Verstelleinrichtung zum positionieren und arretieren des Saugrings im Telezentriebereich des Objektivs,
(5) Feinverstellung zur Positionierung auf dem Messobjekt,
(6) Saugring (siehe auch Abbildung 4),
(7) Vakuum-Drucksensor zum Messen des Drucks im inneren Saugkanal,
(8) Vakuumsaugdüse zum Erzeugen eines Vakuums durch das Ejektor-Prinzip. Die Höhe des Unterdrucks ist abhängig vom Druck der durchströmenden Luft.
(9) Magnetventil zum Abdichten bzw. Belüften des inneren Saugkanals, Das Ventil ist hängend in der Schlauchleitung angebracht.

Die Ausrichtung des Messkopfes Ober dem zu vermessenden Hautareal erfolgt grob über ein Stativ, an dem der Messkopf befestigt ist. Für den Messvorgang wird der Messkopf des HEMG mit dem Saugring (6) auf die Hautoberfläche des Probanden gesetzt, die Ausrichtung ist durch die Feinverstellung (5) möglich, wobei der Anpressdruck auf dem zu untersuchenden Bereich zu minimieren ist.

Die Scharfstellung des Kamerabildes erfolgt durch die Verstelleinrichtung (4), durch die der Abstand zwischen Saugring und Messobjektiv einsteilbar ist.

Die Verstelleinrichtungen (4) und (5) können als Trieb über Zahnstange mittels seitlichem Rändelrad, wie sie insbesondere in optischen Geräten wie Mikroskopen, Kameras eingesetzt werden, ausgeführt werden. Auch der Einsatz von Linearachsen und Spindeltrieben, wie sie im Maschinen- und Instrumentenbau verbreitet sind, ist möglich.

In seinen Grundzügen ist der Aufbau des Messkopfes aus dem Aufbau eines Auflichtmikroskops ableitbar, wobei das Okular im Mikroskop mit der Digitalkamera und der Objekttisch mit dem Saugring vergleichbar sind. Auch hier gibt es Möglichkeiten zur Positionierung und Scharfstellung.

In einer ganz besonders vorteilhaften Ausführung des Messkopfes kann der Druck bzw. die Kraft, mit der der Saugring auf dem zu untersuchenden Areal aufliegt, gemessen werden.

Zur Messung des am Saugring anliegenden Vakuums können kommerziell erhältliche Drucksensoren verwendet werden. Besonders eignen sich Drucksensoren, die für einen Druckbereich von 0 bis -1000 mbar (Vakuum-Drucksensoren) bzw. für einen Druckbereich von 0 bis +1300 mbar (Absolut-Drucksensoren) ausgelegt sind.

Vorteilhaft ist die Verwendung eines Drucksensors, der den Druck im inneren Saugkanal ermittelt, also zwischen Steuerventil und Saugring angeordnet ist.

In einer ganz besonders vorteilhaften Ausführungsform ist durch einen zweiten Drucksensor die Messung des im äußeren Saugkanal herrschenden Drucks möglich.

Abbildungen 4a bis 4b zeigen einen Saugring zur Messung multiaxialer Verschiebungen im Schnitt und in der Draufsicht. Er besteht aus einem schmalen äußeren Saugkanal (61) und einem breiten inneren Saugkanal (62). Die Kanäle sind jeweils durch eine Bohrung mit einem Gewindesackloch (64) verbunden, in das ein Schlauchanschluss (68) geschraubt wird. Die mittlere, durchgehende Bohrung (65) dient der Beleuchtung und der Bildaufnahme.

Wie aus dem Stand der Technik bekannt, ist es möglich, den Schlauchanschlusses auch in anderer Weise auszubilden, z.B. durch Verkleben, Verschweißen, Zusammenstecken oder Verhaken (Bajonettverschraubung).

Der äußere Saugkanal (61) wird zum Fixieren des Messobjekts verwendet. Damit er keine größeren Deformationen am zu untersuchenden Hautareal verursacht, ist er sehr schmal ausgeführt.
Vorzugsweise hat der äußere Saugkanal eine Breite von 0,2 bis 5 mm.

Vorteilhaft kann ein Fixieren des Messobjekts auch über ein Verkleben, insbesondere mit doppelseitigem Klebeband, erfolgen.

Der innere Saugkanal (62) verursacht beim Anlegen eines Unterdrucks durch das Einsaugen von Haut in den Saugkanal (62) eine multiaxiale Dehnung des Messobjekts im Beobachtungsbereich der Kamera.

Abbildung 4c zeigt einen Saugring für die Messung uniaxialer Verschiebungen. Hier ist neben dem äußeren Saugkanal (61), der zur Fixierung dient, kein zweiter (innerer) Saugkanal vorhanden. Die Streckung der Haut erfolgt durch zwei gleichgroße, parallele Saugkammem (66), die symmetrisch um die innere Bohrung (65) angeordnet sind.

Vorzugsweise hat der innere Saugkanal eine Breite von 0,5 bis 10 mm

Der Saugring kann aus allen Materialien gefertigt sein, die bei erfindungsgemäßer Verwendung keiner Formänderung unterliegen, da durch Deformationen die Reproduzierbarkeit der Messergebnisse beeinflusst werden kann.

Die Oberfläche eines erfindungsgemäßen Saugringes sollte vorzugsweise aus einem festen, inerten, vakuumresistenten Material, insbesondere einem Kunststoffmaterial, ganz besonders Teflon, HDPE oder PVC, bestehen.

Ganz: besonders vorteilhaft sind Materialien mit niedriger Wärmekapazität, da sich durch den Temperaturunterschied zwischen Saugring und zu untersuchender Haut Irritationen einstellen können. Ebenso ist eine Temperaturkonditionierung des Saugringes, an einer Stelle die nicht untersucht werden soll, vorteilhaft.

### Verfahren/Auswertung:

Im folgenden sollen das Verfahren sowie das zur Durchführung des Verfahrens vorteilhaft gestaltete Messgerät näher dargelegt werden, ohne die Erfindung damit unnötig einschränken zu wollen.

Das Verfahren zur Messung der elastischen Eigenschaften der Haut, insbesondere der menschlichen Haut, stellt sich wie folgt dar.
Auf die Haut in vivo wird ein mit einer Nut versehener Ring gesetzt, dessen Nut (Saugkanal (62) in Abb. 4) zur Messung der Elastizität/Streckverhaltens evakuiert wird. Dabei wird die Haut in den evakuierten Bereich gezogen. Die Streckung der Haut kann im Innenbereich bildmäßig erfasst werden.

In einer vorteilhaften Ausführung verfügt das vorgestellte HEMG über Applikationsmöglichkeiten, die es gestatten, beispielsweise weitere optische Aufzeichnungen der Hautoberflächenstruktur durchzuführen und zu analysieren oder mittels im Medizinbereich üblicher Ultraschalldiagnostik die Tiefenauswirkung der Auslenkung zu messen. Selbstverständlich ist durch die flexible Einsatzmöglichkeit des HEMG auch die Verwendung anderer, der Geometrie des Messgerätes anpassbarer Meßmethoden möglich, wie beispielsweise die Veränderung des mikrokapillaren Erythrozytenstroms mittels Laser Doppler Flowmetrie.
So weist eine bevorzugte Ausführungsform des Messgeräts, um den Messvorgang so umfassend wie möglich analysieren zu können, zentral eine Öffnung vor, in die die genannten anderen Messgeräte (beispielsweise visuelle Dokumentation mittels Endoskop; Topographieänderungskinetik mittels Lasertriangulationssensoren) eingeführt werden, um weitere Parameter des zu untersuchenden Hautareals zu bestimmen.

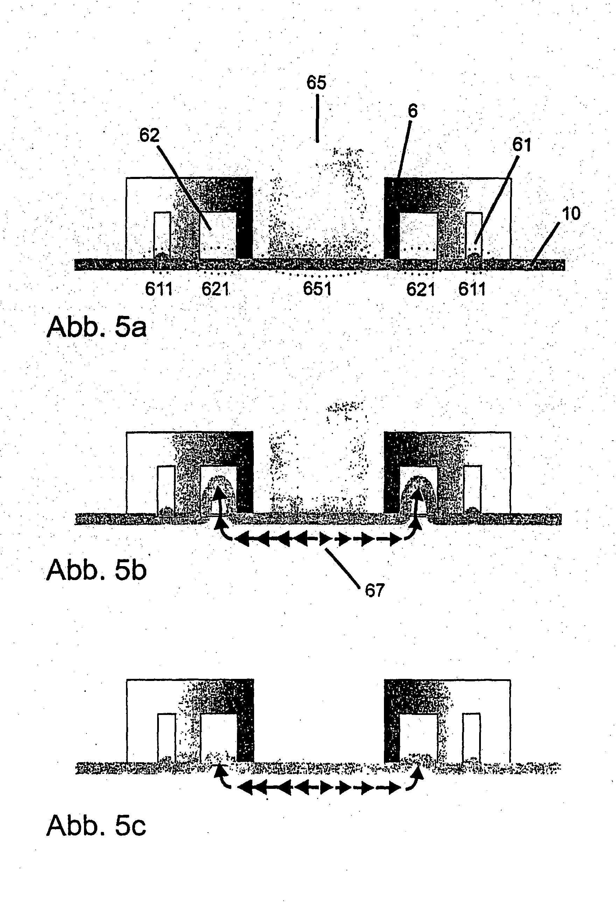

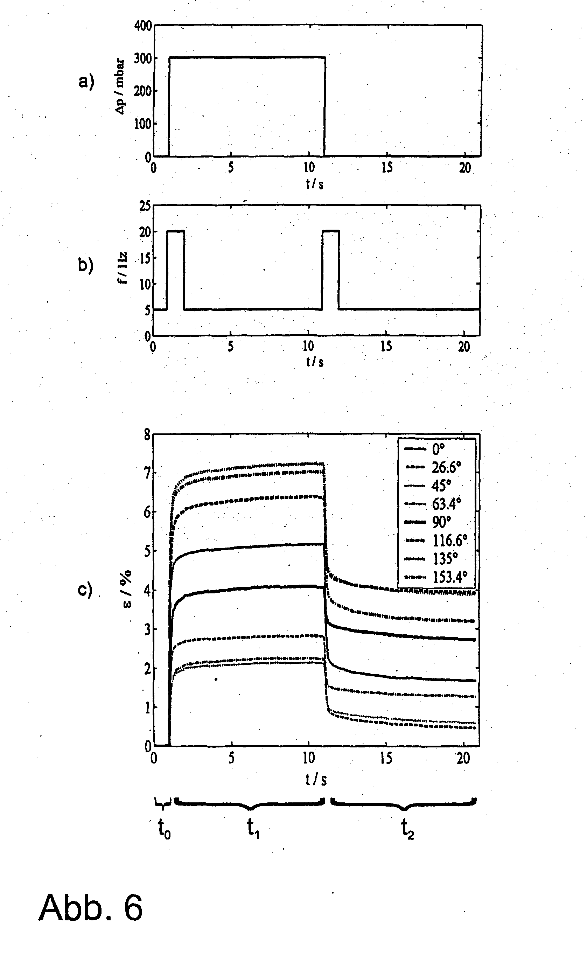

Abbildung 5a zeigt schematisch den auf die Haut (10) aufgesetzten Saugring (6) des HEMG, der durch Anlegen eines Unterdrucks an den äußeren Saugkanal (61) im Bereich (611) auf der Haut fixiert wurde. Dieser Zustand entspricht dem in Abbildung 6 mit t₀ bezeichneten Zeitraum.

Vorzugsweise ist es auch den äußeren und des inneren Saugringes gleichzeitig mit einem Unterdruck zu beaufschlagen. Dadurch ist eine Minimierung der Vorstreckung, der Fehler der durch das Hineinsaugen der Haut in den äußeren Saugkanal auftritt, möglich.

Abbildung 5b zeigt schematisch den Belastungszustand der Haut, der durch Anlegen eines Unterdrucks im inneren Saugkanal (62) ausgelöst wird und zur Folge hat, das Haut in den Saugkanal hineingesaugt wird (Bereich 621) und eine Streckung der Haut im Bereich des Beobachtungsfeldes (651), welches im Zentrum der durchgehenden Bohrung (65) liegt, auslöst. Die dargestellten Pfeile veranschaulichen die infolge der Belastung auftretenden Verschiebungen materieller Punkte der Hautoberfläche. Dieser Zustand entspricht dem in Abbildung 6 mit t₁ bezeichneten Zeitabschnitt.

Abbildung 5c zeigt schematisch den Zustand nach der Vakuumbelastung. Durch mangelnde Hautelastizität erfolgt kein vollständiger Rückzug und eine Restauslenkung verbleibt. Dieser Zustand entspricht dem In Abbildung 6 mit t₂ bezeichneten Zeitabschnitt.

Durch Anlegen eines Vakuums an den inneren Saugkanal des Saugringes wird die Haut des Probanden gedehnt. Während des Dehnungsprozesses werden zeitlich aufgelöst einzelne Photographien durch die CCD Kamera aufgenommen und in der Mess- und Steuereinheit digital gespeichert.

Die Synchronisation von Unterdruckbeaufschlagung des inneren Saugringes und der Bildaufnahme geschieht durch vom Mess- und Auswertesystem generierte Triggerimpulse (s. Abb. 6).

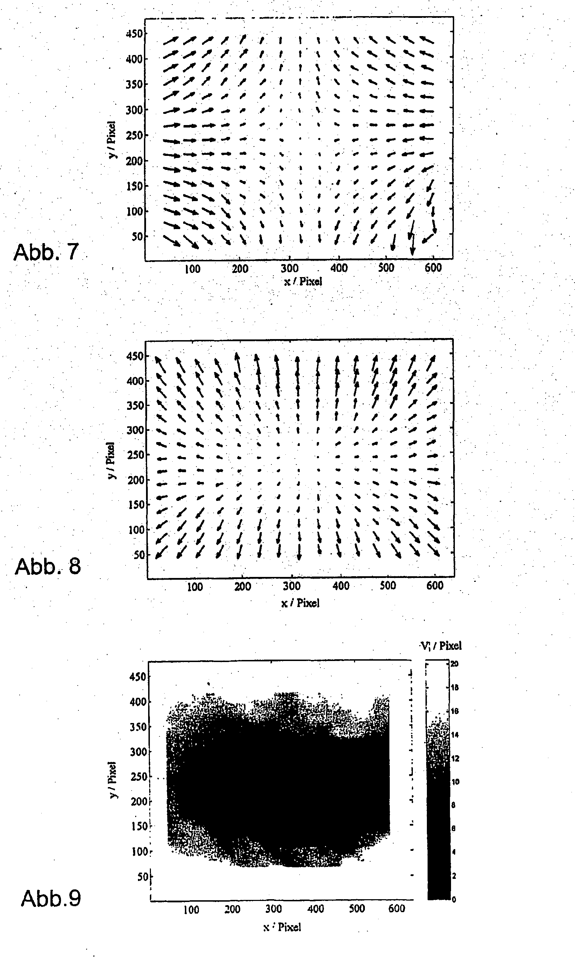

Wird der Unterdruck im inneren Saugring abgebaut, kommt es zu einer Retardationsphase, in der sich die Haut in den Ausgangszustand vor der Dehnung begibt. Schematisch ist die Be- und Entlastung in Abbildung 6 wiedergegeben. Die nach der Entlastung zunächst verbleibende Restauslenkung wird ebenso wie die Auslenkung während der Belastung bildmäßig erfasst.
Aus den so gewonnen Aufnahmen können nach bekannten Methoden der Bildverarbeitung, wie in dem Artikel "Quantification of mechanical properties of human skin in vivo" von Heinrich und Lunderstädt (Proc. of SPIE, Applications of Digital Image Processing XXIV, 4472, pp. 11-20, San Diego, USA, 2001) gezeigt wird, die Verschiebungsvektoren einzelner Hautareale berechnet werden. Dabei werden unter Berücksichtigung eines Ähnlichkeitskriteriums kleine Gebiete aus dem Referenzbild In dem Folgebild wiedergefunden (Template Matching, Mustervergleich) und der Vektor vom Zentrum des Referenz-Musters zum Punkt der besten Übereinstimmung im Folgebild als Verschiebungsvektor definiert. Alle Verschiebungsvektoren zusammen bilden das Verschiebungsvektorfeld. Der Vergleich der Verschiebungsvektoren untereinander liefert Aussagen über Längenänderungen und damit über Dehnungen.
Durch grafische Darstellung der Ergebnisse als winkelabhängige Dehnungskurven (Abbildung 6c) Verschiebungsvektorfeld (Abbildung 7 und 8) oder Graubild (Abbildung 9) sowie durch aus der Mechanik bekannte Berechnungsmethoden (ebener Verzerrungszustand) können Aussagen über Vorzugsrichtungen und Dehnbarkeit der Haut gewonnen werden.

Die Abbildung 7 zeigt ein Verschiebungsvektorfeld, das durch Vergleich zweier Bilder, die vor und während einer multiaxialen Belastung des Messobjekts erfasst wurden, entstanden ist. Die Pfeile geben die skalierten Richtungen der Verschiebungen an. Die tatsächliche Größe der Verschiebung ist durch die Beträge in Abbildung 9 gegeben.

Die Abbildung 8 zeigt ein Verschiebungsvektorfeld im uniaxialen ebenen Zugversuch.

Bevorzugt ist auch die zyklische Be- und Entlastung von Hautarealen.

Die Durchführung der Elastizitätsmessung erfolgt, nachdem der Messkopf auf dem zu untersuchenden Areal positioniert wurde, vorzugsweise nach nachfolgend dargestelltem Ablaufplan:
a) Eingabe der Messparameter,
b) Zwischenspeichern und gegebenenfalls Protokollieren der Messparameter,
c) entsprechendes Umarbeiten der Messparameter in Befehlssequenzen für den Steuerrechner,
d) Abgabe der Steuerbefehle vom Steuerrechner an die Regelventile, bei gleichzeitigem Empfang der Rückmeldung über den angelegten Unterdruck, welcher die Fixierung und Belastung (Dehnung) der Haut auslöst,
e) Abgabe der Triggersignale vom Steuerrechner an die Bilderfassungseinheit,
f) Umwandlung der erfassten Bilder in geeignete Bildformate und Zwischenspeichern der Bilder,
g) Bildverarbeitung:
   - Bildvorverarbeitung,
   - Berechnung der Verschiebungsvektorfelder
h) Auswertung:
   - Berechnung der zeit- und richtungsabhängigen Dehnungsverläufe,
   - Ermittlung der Vorzugsrichtungen (Richtung maximaler/ minimaler Dehnung),
   - Berechnung weiterer Kenngrößen der Dehnungsverläufe
i) Speicherung und Visualisierung der Ergebnisse,
wobei Schritte d) bis f) parallel und/oder wiederholend erfolgen können.

Eine idealer weise interaktive Benutzeroberfläche ermöglicht mindestens die Eingabe von Belastungshöhe und -dauer. Diese Parameter werden von einem Auswerterechner zwischengespeichert, protokolliert und als geeignete Befehlssequenzen an einen Steuerrechner übertragen. Dieser kann beispielsweise als Einschubkarte für den Auswerterechner ausgeführt sein.

Der Steuerrechner erzeugt im weiteren unabhängig vom Auswerterechner die zur Ansteuerung der Ventile (9) benötigten elektrischen Spannungen. Geeignete Algorithmen werten dabei den Druck im inneren Saugkanal aus und führen den Druck so nach, dass die Straffung der Haut dem vom Benutzer geforderten Belastungsdiagramm entspricht.

Die Videosignale der im Gehäuseinnenteil (1) angebrachten CCD-Kamera werden zu vorgegebenen Abtastzeitpunkten, mindestens jedoch zu Beginn und am Ende der Retardationsbewegung durch die Vakuumbeaufschlagung der Haut, durch eine FrameGrabber-Karte dem Auswerterechner digital zur Verfügung gestellt. Durch geeignete Algorithmen werden dann die Vorzugsdehnrichtungen der Haut, d.h. die Richtungen der größten bzw. kleinsten Dehnung, in den Bildsequenzen erkannt und ihre jeweilige Winkellage bestimmt.

### Anwendung:

Das erfindungsgemäße Gerät ist zur Durchführung des erfindungsgemäßen Verfahrens hervorragend geeignet.
Im Vordergrund steht dabei die Bestimmung der Hautelastizität in vivo eines vorher festgelegten Hautareals.

Ist die Hautelastizität des vorgegebenen Hautareals vermessen, lassen sich aus den Ergebnissen die Vorzugsrichtungen der Haut ermitteln. Diese spielen insbesondere bei Operationen eine Rolle, bei denen die Richtung des durchzuführenden Schnittes in weitem Rahmen wählbar ist. Beispielsweise seien hier Operationen genannt, die eine Öffnung der Bauchdecke beinhalten. Oftmals kann hier der Chirurg frei bestimmen, wie er den Schnitt setzen möchte. Setzt er ihn entlang der mittels des Messgerätes zuvor bestimmten Vorzugslinien, ist zu erwarten, dass die sich ergebende Narbe von relativ unscheinbarem Format ist. In einigen Fällen kann insbesondere bei dermatologischen Eingriffen durch geschickte Wahl des Schnitts erreicht werden, dass quasi keine sichtbare Narbe verbleibt.

Weiterhin ermöglicht das Messgerät, auch in schwierigen Anwendungsfällen die Hautelastizität zu bestimmen. So ist für kosmetische Operationen an der Haut in der Nähe des Auges die Bestimmung der herrschenden Elastizitätsverhältnisse in der Haut eigentlich unumgänglich. Die bekannten Testmethoden versagen hier aber vielmals, sei es, dass eine Entnahme einer Hautprobe aufgrund des äußerst empfindlichen Bereiches unterbleiben sollte, sei es, weil die baulich vorgegebene Ausführung des zur Verfügung stehenden Gerätes eine Anwendung in diesem Bereich verhindert.
Dann ist bei entsprechender Ausgestaltung des Messgeräts auch die Vermessung der Elastizität der Kopfhaut möglich, wobei die Kopfhaut zuvor durch eine Rasur von Haaren befreit werden muss.

Vergleicht man die Ergebnisse vor und nach Behandlung mit kosmetischen und/oder dermatologischen Produkten, lassen sich Erkenntnisse über die Wirksamkeit der applizierten Zubereitungen treffen.

Schließlich bietet sich die Verwendung des Messgeräts, an zur zerstörungsfreien Vermessung der Eigenschaften eines elastischen Materials, .insbesondere von Kunststofffolien.

## Patentansprüche

1. Hautelastizitätsmeßgerät (HEMG) enthaltend:
- ein oder mehrere Drucksensoren zur Messung des angelegten Unterdrucks,
- ein oder mehrere elektronische Steuerungs- und Auswertungseinheiten,
- ein oder mehrere Aufzeichnungsgeräte zur Erfassung der Hautbildes
**dadurch gekennzeichnet, dass** es einen Messkopf mit einem Saugring (6) besitzt, der ein oder mehrere Saugnuten (61, 62) zur Be- und Endlastung der Haut mittels Unterdruck und eine mittlere, zentrale Bohrung zur Beobachtung des zu untersuchenden Hautareals aufweist.

2. HEMG nach Anspruch 1, **dadurch gekennzeichnet, dass** der Messkopf mit Hilfe von Unterdruck auf der Haut fixiert wird.

3. HEMG nach Anspruch 1, **dadurch gekennzeichnet, dass** der Messkopf mit Hilfe eines Klebstoffes oder Klebebandes auf der Haut fixiert wird.

4. HEMG nach mindestens einem der vorhergehenden Ansprüche, **dadurch gekennzeichnet, dass** die Saugnut ringförmig ist.

5. HEMG nach mindestens einem der vorhergehenden Ansprüche, **dadurch gekennzeichnet, dass** der Messkopf mindestens zwei Saugnuten aufweist, die symmetrisch um die mittlere, zentrale Bohrung angeordnet sind.

6. HEMG nach mindestens einem der vorhergehenden Ansprüche, **dadurch gekennzeichnet, dass** das Aufzeichnungsgerät eine Digitalkamera ist, insbesondere eine Progressive Scan Kamera.

7. HEMG nach mindestens einem der vorhergehenden Ansprüche, **dadurch gekennzeichnet, dass** die elektronische Steuerungs- und Auswerteeinheit ein geeigneter Personalcomputer ist.

8. HEMG nach mindestens einem der vorhergehenden Ansprüche, **dadurch gekennzeichnet, dass** die Bestrahlung des Messfeldes durch eine Strahlungsquelle erfolgt, insbesondere durch eine Kaltlichtlampe.

9. HEMG nach mindestens einem der Vorhergehenden Ansprüche, **dadurch gekennzeichnet, dass** dem Aufzeichnungsgerät eine Optik vorgeschaltet ist.

10. HEMG nach mindestens einem der vorhergehenden Ansprüche, **dadurch gekennzeichnet, dass** im Saugring eine zentrale Öffnung vorgesehen ist, in die Messgeräte eingeführt werden können, um weitere Parameter des zu untersuchenden Hautareals zu bestimmen.

11. HEMG nach mindestens einem der vorhergehenden Ansprüche, **dadurch gekennzeichnet, dass** die Steuereinheit und die Auswerteinheit an verschiedenen Orten lokalisiert sind und/oder separate für sich austauschbare Einheiten bilden.

12. Verfahren zur Durchführung einer in vivo Etastizitätsmessung mit einem HEMG nach mindesten einem der Ansprüche 1 bis 11, **dadurch gekennzeichnet, dass** auf die Haut ein mit einem Saugkanal versehener Ring gesetzt wird, der zur Messung der Elastizität/Streckverhaltens evakuiert wird, wobei die Haut in den evakuierten Bereich gezogen wird und die Streckung der Haut im Innenbereich bildmäßig erfasst werden kann,
wobei folgende Schritte abgearbeitet werden:
a) Eingabe der Messparameter und Applikation des Meßkopfes auf dem zu untersuchenden Areal,
b) Zwischenspeichern und gegebenenfalls Protokollieren der Messparameter,
c) entsprechendes Umarbeiten der Messparameter in Befehlssequenzen für den Steuerrechner,
d) Abgabe der Steuerbefehle vom Steuerrechner an die Regelventile bei gleichzeitigem Empfang der Rückmeldung über den angelegten Unterdruck,
e) Abgabe der Triggersignale vom Steuerrechner an das Aufzeichnungsgerät,
f) Umwandlung der erfassten Bilder in geeignete Bildformate und Zwischenspeichern der Bilder,
g) Bildverarbeitung:
- Bildvorverarbeitung,
- Berechnung der Verschiebungsvektorfelder
h) Auswertung:
- Berechnung der zeit- und richtungsabhängigen Dehnungsverläufe,
- Ermittlung der Vorzugsrichtungen (Richtung maximaler/ minimaler Dehnung),
- Berechnung weiterer Kenngrößen der Dehnungsverläufe
i) Speicherung und Visualisierung der Ergebnisse,
wobei Schritte d) bis f) parallel und/oder wiederholend erfolgen können.

13. Verwendung eines HEMG nach mindestens einem der vorhergehenden Ansprüche 1 bis 11 zur zerstörungsfreien Vermessung der Eigenschaften der Haut, insbesondere der menschlichen Haut.

14. Verwendung eines HEMG nach mindestens einem der vorhergehenden Ansprüche 1 bis 11 zur zerstörungsfreien Bestimmung der Eigenschaftsänderungen der Haut, insbesondere der menschlichen Haut, vor und nach der Applikation von dermatologischen und/oder kosmetischen Zubereitungen.

15. Verwendung eines HEMG nach mindestens einem der vorhergehenden Ansprüche 1 bis 11 zur zerstörungsfreien Vermessung der Eigenschaften eines elastischen Materials, insbesondere von Kunststofffollen.

## Claims

1. Device for measuring skin elasticity (HEMG) comprising:
- one or a plurality of pressure sensors for measuring the vacuum applied,
- one or a plurality of electronic control and measuring units,
- one or a plurality of recording devices for capturing a picture of the skin,
**characterized in that** it comprises a measuring head with a suction ring (6) having one or a plurality of suction grooves (61, 62) for loading and relaxing the skin using a vacuum and a middle central bore for observing the skin area to be examined.

2. HEMG of claim 1, **characterized in that** the measuring head is fixed on the skin by means of a vacuum.

3. HEMG of claim 1, **characterized in that** the measuring head is fixed on the skin by means of glue or adhesive tape.

4. HEMG of at least one of the preceding claims, **characterized in that** the suction groove is annular in shape.

5. HEMG of at least one of the preceding claims, **characterized in that** the measuring head comprises at least two suction grooves symmetrically arranged about the middle central bore.

6. HEMG of at least one of the preceding claims, **characterized in that** the recording device is a digital camera, especially a progressive scan camera.

7. HEMG of at least one of the preceding claims, **characterized in that** the electronic control and evaluating unit is a suitable personal computer.

8. HEMG of at least one of the preceding claims, **characterized in that** the measuring field is irradiated by means a radiation source, especially a cold-light lamp.

9. HEMG of at least one of the preceding claims, **characterized in that** an optic is disposed upstream of the recording device.

10. HEMG of at least one of the preceding claims, **characterized in that** a central opening is provided in the suction ring, into which measuring devices may be inserted to determine further parameters of the skin area to be examined.

11. HEMG of at least one of the preceding claims, **characterized in that** the control unit and the evaluating unit are located at different places and/or form separate units adapted to be replaced individually.

12. Method for performing an in-vivo elasticity measurement using a HEMG of at least one of claims 1 to 11, **characterized in that** a ring with a suction channel is applied to the skin, the ring being evacuated to measure the elasticity/stretching behaviour of the skin, wherein the skin is drawn into the evacuated area so that the stretching of the skin can be captured as a picture on the inside thereof, the following steps being performed:
a) inputting the measuring parameters and applying the measuring head to the area to be examined,
b) latching and possibly recording the measuring parameters,
c) correspondingly transforming the measuring parameters into instruction sequences for the controlling computer,
d) outputting the control instructions from the controlling computer to the control valves with a simultaneous reception of a feedback on the vacuum applied,
e) outputting trigger signals from the control computer to the recording device,
f) transforming the captured pictures into suitable picture formats and latching the pictures,
g) picture processing:
- picture pre-processing,
- calculating the displacement vector field,
h) evaluation:
- calculating the time- and direction-dependent stretching characteristics,
- determining the preferred directions (direction of maximum/minimum stretching),
- calculating further parameters of the stretching characteristics,
i) storing and visualizing the results,
wherein the steps d) to f) may be performed in parallel and/or repeatedly.

13. Use of a HEMG of at least one of the preceding claims 1 to 11 for a nondestructive measuring of the properties of skin, especially of human skin.

14. Use of a HEMG of at least one of the preceding claims 1 to 11 for a nondestructive determination of the changes in the properties of skin, especially of human skin, before and after the application of dermatologic and/or cosmetic preparations.

15. Use of a HEMG of at least one of the preceding claims 1 to 11 for a nondestructive measuring of the properties of an elastic material, especially of plastic materials.

## Revendications

1. Appareil de mesure de l'élasticité de la peau (HEMG) contenant :
- un ou plusieurs capteurs de pression pour la mesure de la dépression appliquée,
- une ou plusieurs unités électroniques de commande et d'exploitation,
- un ou plusieurs appareils d'enregistrement pour l'enregistrement de l'image de la peau,
**caractérisé en ce qu'**il possède une tête de mesure dotée d'une bague d'aspiration (6) qui comporte une ou plusieurs gorges d'aspiration (61, 62) pour augmenter et réduire la dépression appliqué sur la peau et un perçage central médian pour l'observation de la surface de la peau à examiner.

2. HEMG selon la revendication 1, **caractérisé en ce que** la tête de mesure est fixée sur la peau par dépression.

3. HEMG selon la revendication 1, **caractérisé en ce que** la tête de mesure est fixée sur la peau à l'aide d'un adhésif ou d'une bande adhésive.

4. HEMG selon au moins l'une des revendications précédentes, **caractérisé en ce que** la gorge d'aspiration est annulaire.

5. HEMG selon au moins l'une des revendications précédentes, **caractérisé en ce que** la tête de mesure comporte au moins deux gorges d'aspiration qui sont disposées symétriquement autour du perçage central médian.

6. HEMG selon au moins l'une des revendications précédentes, **caractérisé en ce que** l'appareil d'enregistrement est une caméra numérique, en particulier une caméra Progressive Scan.

7. HEMG selon au moins l'une des revendications précédentes, **caractérisé en ce que** l'unité électronique de commande et d'exploitation est un ordinateur personnel approprié.

8. HEMG selon au moins l'une des revendications précédentes, **caractérisé en ce que** le rayonnement du champ de mesure est effectué par une source de rayonnement, en particulier par une lampe à lumière froide.

9. HEMG selon au moins l'une des revendications précédentes, **caractérisé en ce qu'**un dispositif optique est monté en amont de l'appareil d'enregistrement.

10. HEMG selon au moins l'une des revendications précédentes, **caractérisé en ce qu'**il est prévu dans la bague d'aspiration une ouverture centrale dans laquelle des appareils de mesure peuvent être introduits pour déterminer d'autres paramètres de la surface de la peau à examiner.

11. HEMG selon au moins l'une des revendications précédent, **caractérisé en ce que** l'unité de commande et l'unité d'exploitation sont placées en différents endroits et/ou forment des unités séparées remplaçables en soi.

12. Procédé de réalisation d'une mesure d'élasticité in vivo au moyen d'un HEMG selon au moins l'une des revendications 1 à 11, **caractérisé en ce qu'**une bague dotée d'un canal d'aspiration est placée sur la peau et est soumis à une dépression pour effectuer la mesure de l'élasticité ou du comportement en extension, la peau étant étirée dans la région soumise à une dépression et l'extension de la peau pouvant être mesurée sous forme d'image dans la région intérieure, les étapes suivantes étant réalisées :
a) entrée des paramètres de mesure et application de la tête de mesure sur la surface à examiner,
b) mémorisation temporaire et éventuellement consignation des paramètres de mesure,
c) transformation appropriée des paramètres de mesure en séquence d'instructions pour l'ordinateur de commande,
d) délivrance des instructions de commande par l'ordinateur de commande à des clapets de régulation lors de la réception simultanée d'un signal de retour sur la dépression appliquée,
e) délivrance des signaux de déclenchement par l'ordinateur de commande à l'appareil d'enregistrement,
f) conversion des images acquises en formats d'image appropriés et mémorisation temporaire des images,
g) traitement d'image,
- prétraitement d'image,
- calcul des champs de vecteurs de déplacement
h) exploitation :
- calcul des variations d'extension en fonction du temps et de la direction,
- détermination des directions prédominantes (direction d'extension maximale/minimale),
- calcul d'autres grandeurs caractéristiques des variations d'extension,
i) mémorisation et visualisation des résultats, les étapes d) à f) pouvant être effectuées en parallèle et de façon répétée.

13. Utilisation d'un HEMG selon au moins l'une des revendications précédentes 1 à 11 pour mesurer sans destruction les propriétés de la peau, en particulier de la peau humaine.

14. Utilisation d'un HEMG selon au moins l'une des revendications précédentes 1 à 11 pour déterminer sans destruction les variations de propriété de la peau, en particulier de la peau humaine, avant et après l'application de préparations dermatologiques et/ou cosmétiques.

15. Utilisation d'un HEMG selon au moins l'une des revendications précédentes 1 à 11 pour la mesure sans destruction des propriétés d'un matériau élastique, en particulier de feuilles de matière plastique.
